# EUROPEAN PATENT APPLICATION

(11) **EP 2 535 412 A1**
(43) Date of publication of application: **19.12.2012**
(21) Application number: 11382207.6
(22) Date of filing: 17.06.2011
(51) Int. Cl.: C12N 15/113, A61K 31/713, A61K 48/00, A61P 21/00

(54) **New treatment for muscular dystrophies**

(71) Applicant: Universitat Pompeu-Fabra, 08002 Barcelona (ES); Institució Catalana de Recerca i Estudis Avançats, 08018 Barcelona (ES)
(72) Inventor: Munoz-Cánoves, Purificación, 08003 Barcelona (ES); Raya Chamorra, Marina, 08003 Barcelona (ES); Perdiguero Santamaria, Eusebio, 08003 Barcelona (ES); Luis Serrano, Antonio, 08003 Barcelona (ES)
(74) Representative: ZBM Patents

(57) **Abstract**

The invention provides a miRNA-21 inhibitor and pharmaceutical compositions comprising said miRNA-21 inhibitor together with pharmaceutical excipients and carriers for use in the treatment of a muscular dystrophy and/or a muscular dystrophy-like myopathy. Particularly, the miRNA-21 inhibitors and pharmaceutical compositions comprising the same are for use in the regeneration of skeletal muscle in muscular dystrophy and/or a muscular dystrophy-like myopathy patients.

SEQUENCE LISTING

<110>  Universitat Pompeu Fabra
       Institució Catalana De Recerca i Estudis Avançats

<120>  New treatment for muscular dystrophies

<130>  P2003EP00

<160>  1

<170>  PatentIn version 3.5

<210>  1
<211>  22
<212>  RNA
<213>  Homo sapiens

<400>  1
uagcuuauca gacugauguu ga                                                22

## Description

The present invention is related to the field of medicine and, specially, the field of muscle disorders or myopathies. More particularly, the invention is related to new agents for the treatment of muscular dystrophies.

### BACKGROUND ART

Muscular dystrophy (from now on abbreviated "MD") refers to a group of inherited myopathies that weakens the muscles that move the human body. MDs are characterized by progressive skeletal muscle weakness, defects in muscle proteins, and the death of muscle cells and tissue. Diseases including Duchenne, Becker, limb girdle, congenital, facioscapulohumeral, myotonic, oculopharyngeal, distal, and Emery-Dreifuss are always classified as MD but there are more than 100 diseases in total with similarities to MD. Most types of MD are multi-system disorders with manifestations in body systems including the heart, gastrointestinal and nervous systems, endocrine glands, skin, eyes and even brain. The condition may also lead to mood swings and learning difficulties.

Duchenne muscular dystrophy (from now on "DMD") is the most common genetic muscle disease affecting 1 in 3,500 live male births. It is an X-linked recessive disease caused by a defective dystrophin gene. Dystrophin is located beneath the sarcolemna, assembles the dystrophin-glycoprotein complex at the sarcolemna, and links the internal cytoplasmic actin filament network and extracellular matrix, providing physical strength to muscle fibers. Loss of dystrophin in DMD patients results in progressive muscle weakness, wasting and degeneration.

At present, there is no effective therapy to stop the lethal progression of MDs, although several experimental strategies are currently under investigation. Most of these strategies focus on either replacing or compensating for dystrophin. These include vector-mediated gene delivery and exon skipping.

The aim of vector-mediated gene delivery is to provide an alternative copy of the functional dystrophin gene for patients rather than repair the locus within the patient's genome. An advantage of gene replacement for DMD is that it is not dependent on any particular mutation in a patient's gene. However identifying safe and efficient methods to deliver a replacement dystrophin or dystrophin-like gene to muscles body-wide is a daunting challenge. Results from recent studies highlight immunological complications associated with viral vector-mediated gene transfer. Further, longterm expression of dystrophin in muscles throughout the body is a major barrier to clinical success.

Antisense-induced exon-skipping strategies, which aim to remove the mutated or additional exon(s) to restore the reading frame, have been shown to induce the expression of 'Becker MD-like' shortened forms of dystrophin protein. However, several problems limit this approach, including the poor cellular uptake and relative rapid clearance from circulation, but also the high variability in exon-skipping efficiency among muscle types. Experiments in animal models demonstrated that large doses over several weeks were required for functional improvement but not enough to achieve uniform expression of dystrophin. Additionally, the different deletions that cause DMD would require skipping of different exons, and therefore design and optimization of many specific antisense oligonucleotides. If each antisense oligonucleotide is considered a new drug, as currently considered by the Food and Drug Administration (FDA), then each of them will have to go through the expensive and lengthy clinical trial stages. On top of being an insuperable barrier in terms of money and time, it might be very problematic to find enough patients to even perform clinical trials. Additionally, antisense-induced exon-skipping strategies theraphies could be effective in preventing or retarding onset of DMD by targeting the dysfunctional dystrophin molecule but they have, however, little effect on advanced disease.

Other approaches are directed to ameliorating muscle fibrosis, which is a prominent pathological feature of DMD. Human patients with DMD suffer progressive muscle fiber degeneration, the dystrophic fibers being gradually replaced by fibrotic infiltrates. This fibrotic process enhances muscle dysfunction and contributes to the lethal DMD phenotype. It has been proposed that delivery of agents decreasing fibrin accumulation may constitute a novel therapeutic alternative for DMD treatment. This approach could be useful to prevent greater muscle damage, especially when applied early during disease progression. Anti-fibrotic therapies cannot, however, prevent skeletal muscle fiber degeneration, let alone induce muscle regeneration. Thus, muscle dysfunction, once occurred, cannot be in any way reversed.

It is clear that, despite the advances made in this field, there is still a need for alternative therapies that are more effective in the treatment of muscular dystrophies.

### SUMMARY OF THE INVENTION

It has been surprisingly found that negatively regulating miRNA-21 (miR-21) expression in a mouse model with DMD (*mdx* mouse) promotes skeletal muscle regeneration and ameliorates fibrosis.

Thus, a first aspect of the invention provides a miR-21 inhibitor for use in the treatment of a muscular dystrophy and/or a muscular dystrophy-like myopathy. This aspect can be reformulated as the use of a miR-21 inhibitor for the manufacture of a medicament for the treatment of a muscular dystrophy and/or a muscular dystrophy-like myopathy. The invention also provides a method for the treatment of a muscular dystrophy and/or a muscular dystrophy-like myopathy which comprises administering a miR-21 inhibitor to an animal, including a human, in need thereof.

In the sense of the present invention the term "treatment" is referred to the administration of an agent aimed to ameliorate the cause or the symptoms of muscular dystrophy disease but also to avoid the onset of such symptoms. Thus for the scope of the present invention the term "treatment" also includes the concept of prevention.

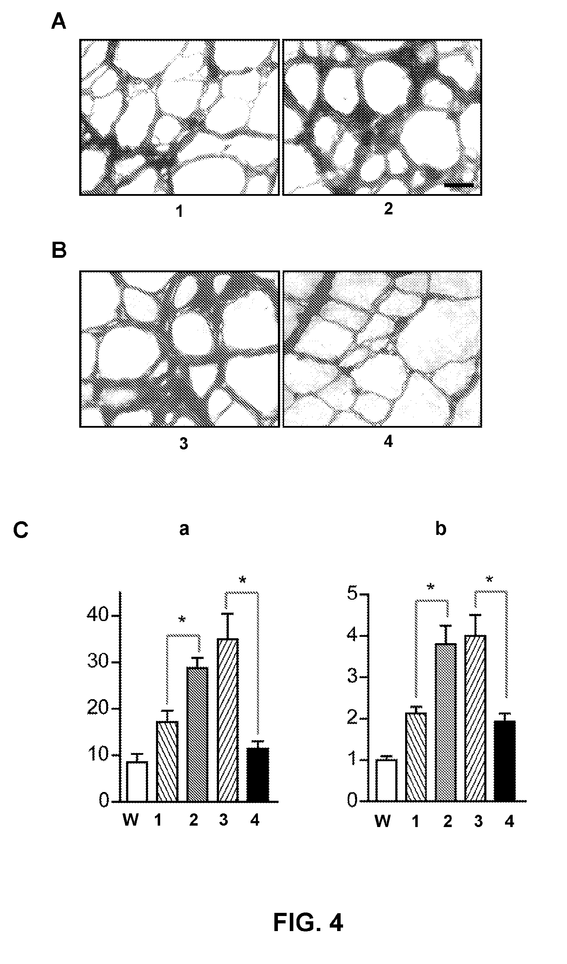

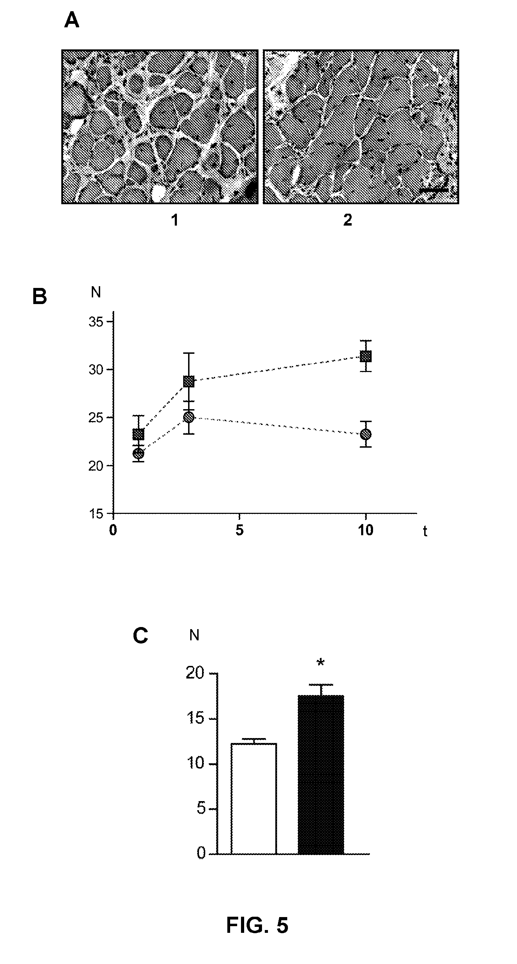

As shown in the examples below, the administration of an agent that reduces miR-21 expression is highly effective in preventing fibrosis in aged *mdx* mice, as well as in reversing muscular damage due to enhanced muscular fiber regeneration (FIGs. 4-5). The effects of the miR-21 inhibitor in muscle regeneration are particularly remarkable in that the aged mice partially recovered muscle functionality (FIG. 5B-C).

The invention has the advantage that the patient suffering from a muscular dystrophy can benefit from the treatment both at an early stage of the disease, to avoid muscular damage, as well as at a later stage of the disease, to recover muscle functionality.

The miR-21 inhibitor as defined in the first aspect of the invention can be administered in the form of a pharmaceutical composition comprising an effective amount of said miR-21 inhibitor together with pharmaceutically acceptable excipients and/or carriers.

Therefore, a second aspect of the invention relates to a pharmaceutical composition for use in the treatment of a muscular dystrophy and/or a muscular dystrophy-like myopathy, comprising a miR-21 inhibitor together with pharmaceutically acceptable excipients and/or carriers. This aspect may be reformulated as the use of a pharmaceutical composition comprising a miR-21 inhibitor together with pharmaceutically acceptable excipients and/or carriers for the manufacture of a medicament for the treatment of a muscular dystrophy and/or a muscular dystrophy-like myopathy. The invention also provides a method for the treatment of a muscular dystrophy and/or a muscular dystrophy-like myopathy which comprises administering to an animal, including a human, in need thereof a pharmaceutical composition comprising a miR-21 inhibitor together with pharmaceutically acceptable excipients and/or carriers.

### DETAILED DESCRIPTION OF THE INVENTION

The present invention provides a miR-21 inhibitor for the treatment of MD or related myopaties.

Skeletal muscle is a form of striated muscle tissue, which is made up of individual components known as muscle fibers. In the sense of the present invention, the term "muscle" refers to multiple bundles of skeletal muscle fibers held together by connective tissue, the term "muscle tissue" is referred to skeletal muscle tissue and the term "fiber" is referred to skeletal muscle fiber.

Muscular dystrophy (or MD) refers to a group of diseases that cause weakness and progressive degeneration of skeletal muscles. There are different forms of muscular dystrophy which differ in their mode of inheritance, age of onset, severity and pattern of muscles affected. The most well-known muscular dystrophies are Duchenne, Becker, limb girdle, congenital, facioscapulohumeral, myotonic, oculopharyngeal, distal, Miyoshi myopathy and Emery-Dreifuss but there are more than 100 myopathies with similarities to muscular dystrophy, all of which are included within the scope of the present invention under the term "muscular dystrophy-like myopathies". The term "myopathy" refers to a muscular disease in which the skeletal muscle fibers do not function for any one of many reasons, resulting in muscular weakness.

DMD has been often considered as a model muscular dystrophy. DMD results from mutations in the gene coding for the protein dystrophin, which localizes at the innerface of the sarcolemma. Dystrophin associates with a large complex of membrane proteins, called the dystrophin glycoprotein complex, important for cell membrane integrity. Without the dystrophin complex to tether the actin cytoskeleton inside the muscle cell to the extracellular matrix, forces generated by the muscle fiber result in tears of sarcolemma leading to muscle damage. The *mdx* mice strain is the most widely used animal model for DMD, having a nonsense mutation in exon 23 which eliminates dystrophin expression. Human patients with DMD and *mdx* mice suffer progressive skeletal muscle degeneration.

Muscle degeneration is a common feature of MD patients. Skeletal fiber loss is initially compensated by proliferation and fusion with preexisting fibers of satellite cells, resulting in an increase in muscle size. After repetitive cycles of muscle degeneration and regeneration, the dystrophic muscle damage can, however, ultimately not be repaired anymore and the dystrophic fibers become gradually replaced, initially by fibrotic infiltrates and subsequently by fat tissue. In fact, muscles of DMD patients or *mdx* mice, as well as other MD patients, present high fibrosis. The whole degenerative process leads to loss of normal muscle function.

The inventors have found that skeletal muscle tissue dysfunction in MD is related to the disregulation of miR-21. It has also been surprisingly found that down regulation of miR-21 effectively enhances tissue regeneration and ameliorates fibrosis. The examples below show that miR-21 interference for 1 month in very old *mdx* dystrophic mice reversed muscle deterioration. The results show not only an attenuation of fibrosis but also a surprising recovery of skeletal muscle (FIG. 4-5). Unexpectedly, old *mdx* dystrophic mice recovered muscle functionality after miR-21 inhibitoty treatment (FIG. 5 B-C).

Thus in a particular embodiment of the first aspect of the invention, a miR-21 inhibitor is provided for use in the treatment of a MD or a related myopathy, wherein the treatment comprises the regeneration of skeletal muscle. The function of miR-21 has been classically associated with cancer based on its high expression in most human tumors, but no role for miR-21 has yet been disclosed in muscle repair.

Preferably, the MD to be treated is selected from the group consisting of Duchenne muscular dystrophy, Becker's muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy. In a particular embodiment, the treatment provided by the invention is for Duchenne muscular dystrophy.

Muscle degeneration/regeneration mechanism as defined above is believed to occur not only as a result of a MD or MD-like myopaty, but also as a result of a different type of disease or an injury. Inhibition of miR-21 by means of a miR-21 inhibitor on muscle regeneration is thus effective in the treatment of muscle diseases related to defective skeletal muscle regeneration.

microRNAs (miRNAs) are short (20-24 nt) non-coding RNAs that are involved in post-transcriptional regulation of gene expression in multicellular organisms by affecting both the stability and translation of mRNAs. miRNAs are transcribed by RNA polymerase II as part of capped and polyadenylated primary transcripts (pri-miRNAs) that can be either protein-coding or non-coding. The primary transcript is cleaved by the Drosha ribonuclease III enzyme to produce an approximately 70-nt stem-loop precursor miRNA (pre-miRNA), which is further cleaved by the cytoplasmic Dicer ribonuclease to generate the mature miRNA and antisense miRNA star (miRNA*) products. The mature miRNA is incorporated into a RNA-induced silencing complex (RISC), which recognizes target mRNAs through imperfect base pairing with the miRNA and most commonly results in translational inhibition or destabilization of the target mRNA. Recent studies have shown that expression levels of numerous miRNAs are associated with various cancers. The modulation of these miRNAs, for instance, by RNA interference technology, thus represents a promising approach for cancer therapy.

miR-21 is a mammalian miRNA that is encoded by the MIR21 gene. miR-21 was one of the first mammalian miRNAs identified. The mature miR-21 sequence is strongly conserved throughout evolution. The human miRNA-21 gene is located on plus strand of chromosome 17q23.2 (55273409-55273480) within a coding gene TMEM49 (also called vacuole membrane protein). Despite being located in intronic regions of a coding gene in the direction of transcription, it has its own promoter regions and forms a ~3433-nucleotides long primary transcript of miR-21 (known as pri-miR-21) which is independently transcribed. The stem-loop precursor of miR-21 (pre-miR-21) resides between nucleotides 2445 and 2516 of pri-miR-21. The predicted stem-loop precursor of human miR-21 is designated by Genbank Accession No. NR_029493 (updated 05-June-2011). Pri-miR-21 is cut by the endonuclease Drosha in the nucleus to produce pre-miR-21, which is exported into the cytosol. This pre-miR-21 is then processed to mature miR-21 (SEQ ID NO: 1). Mature miR-21 is then loaded into miRNA ribonucleoprotein complex RISC and guided to target mRNAs with near perfect complimentarily at 3'UTR.

In addition to its association with cancer, miR-21 has been reported as one of ten different miRNAs showing distinct expression patterns in MD (Eisenberg I, et al. "Distinctive patterns of miRNA expression in primary muscular disorders". Proceedings of the National Academy of Sciences (2007), vol. 104, p. 17016-17021). Additionally, patent application EP2258863 discloses miR-21 as a biomarker for the diagnosis of DMD.

The miR-21 inhibitor according to the present invention is a compound that can reduce or prevent the transcription of miR-21. Since miR-21 is a regulatory micro-RNA, an effective miR-21 inhibitor according to the present invention includes a compound that can partially or completely prevent miR-21 regulatory activity. Partial or complete inhibition of miR-21 regulatory activity may succeed, for example, by avoiding the formation of RNA-induced silencing complex or directly blocking regulatory sites in the target gene, i.e. a gene that is regulated by miR-21. Any other mechanism of miRNA inhibition known in the art is encompassed in the present invention.

In a particular embodiment of the present invention, the miR-21 inhibitor is selected from the group consisting of an inhibitory oligonucleotide, a protein, a ribozyme, a small molecule chemical compound, and a combination thereof. Additionally, the invention contemplates compounds that may inhibit the expression or activity of any downstream signaling molecule of miR-21.

Preferably, inhibition of miR-21 is achieved by administering inhibitory oligonucleotides. The term "inhibitory oligonucleotide" generally refers to an oligonucleotide that may reduce miR-21 transcription or regulatory activity. It is thus a type of miR-21 inhibitor. An oligonucleotide is a polymer having two or more deoxyribonucleotides, ribonucleotides or nucleotide analog molecules. Further, the inhibitory oligonucleotides of the invention may have at least one chemical modification. They may be comprised of one or more locked nucleic acids (LNAs), which are modified ribonucleotides that contain an extra bridge between the 2' and 4' carbons of the ribose sugar moiety resulting in a locked conformation that confers enhanced thermal stability to oligonucleotides containing the LNAs. Alternatively, the inhibitory oligonucleotides may comprise peptide nucleic acids (PNAs), which contain a peptide-based backbone rather than a sugar-phosphate backbone. Other chemical modifications that the oligonucleotides may contain include, but are not limited to, sugar modifications, such as T- O-alkyl (e.g. 2'-O-methyl, 2'-O-methoxyethyl), 2'-fluoro, and 4' thio modifications, and backbone modifications, such as one or more phosphorothioate, morpholino, or phosphonocarboxylate linkages. Other modifications to enhance stability and improve efficacy of oligonucleotides, such as those described in U.S. Patent No. 6,838,283, are known in the art and are suitable for use in the methods of the invention.

Such oligonucleotide inhibitors can be designed and synthesized by techniques known in the art. Often, synthetic miRNA inhibitor designs incorporate the reverse complement of the mature miRNA (the target site) and are chemically modified to enhance binding affinity and provide resistance to nucleolytic degradation. When delivered to a cell, binding of endogenous mature miRNAs to the inhibitor's complementary synthetic target sites is thought to be irreversible, thus these inhibitors are presumed to sequester the endogenous miRNA, making it unavailable for normal function. Inhibitory oligonucleotides for miR-21 are commercially available.

Preferably, the inhibitory oligonucleotide according to the invention targets the mature miR-21 sequence (SEQ ID NO: 1), but it can also target pre-miR-21.

In some embodiments, inhibitory oligonucleotides comprise a sequence that is at least partially or substantially complementary to miR-21 sequence. In the sense of the present invention, an oligonucleotide which is "partially complementary" to miR-21 means that the oligonucleotide is about 75%, 80%, 85%, 90%, 95%, 96%, 97%, 98%, or 99% complementary to said miR-21 sequence. By "substantially complementary" it is meant that the oligonucleotide is about 95%, 96%, 97%, 98%, or 99% complementary to miR-21 sequence. In another embodiment, the oligonucleotide comprises a sequence that is 100% complementary to miR-21. In a preferred embodiment, the oligonucleotide comprises a sequence that is 100% complementary to SEQ ID NO: 1.

Preferably, the miR-21 inhibitory oligonucleotide for use in the present invention is a small single stranded RNA, a small hairpin RNA (shRNA) or a small interference RNA (siRNA).

In a particular embodiment, the miR-21 inhibitory oligonucleotide is a small single stranded RNA oligonucleotide, preferably with modified bases (i.e. 2'-OH-methyl ribose analogs), against miR-21 (also known as mirmask). These small single stranded RNAs are at least partially complementary, but preferably, substantially complementary, or 100% complementary, to the miR-21 sequence and they often have mispairing at the cleavage site of Ago2 or some sort of base modification to inhibit Ago2 cleavage. Argonaute 2 (or Ago2) is a protein involved in RISC function and is essential for miRNA-induced silencing. Small single stranded RNA oligonucleotides may also comprise one or more phosphorothioate linkages resulting in a partial or full phosphorothioate backbone.

In another particular embodiment, the miR-21 inhibitory oligonucleotide is a double stranded or partially double stranded RNA, such as a small interfering RNA (siRNA) or a short hairpin RNA (shRNA). In a prefered embodiment, the miR-21 inhibitor is a shRNA. miR-21 hairpin inhibitors are fully 2'-O-methylated molecules containing the reverse complement sequence of the mature miR-21 (SEQ ID NO: 1) flanked on both sides by hairpin sequences. The manufacture of shRNAs against a specific miRNA is known in the state of the art. Hairpins are usually formed by polypyrimidine (8Yhp + RC + 8Yhp) sequences and consist of an 8 base pair (bp) stem associated with a tetranucleotide loop (Vermeulen A, et al. "Double-stranded regions are essential design components of potent inhibitors of RISC function". RNA, 2007, vol. 13, p. 723-30. Robertson B, et al. "Specificity and functionality of miRNA inhibitors". Silence, 2010, vol. 1, p. 10). When delivered to a cell, binding of endogenous mature miRNAs to these complementary synthetic target sites is irreversible, thus these inhibitors sequester the endogenous miRNA, blocking its function in RISC. shRNAs against miR-21 are commercially available.

To facilitate *in vivo* delivery and stability, the inhibitory oligonucleotides may be linked to a cholesterol or other stabilizing and/or delivery moiety at its 3' end.

RNA based compounds for therapeutic treatment can be chemically synthesized and further administered to the organism. Alternatively, they can be administered as expression vectors, thus being the cell that synthesizes the RNA based compound. The term "vector" in the present invention refers to a composition of matter which can be used to deliver a nucleic acid of interest to the interior of a cell. Non-limiting examples of vectors include an autonomously replicating plasmid or a virus. For purposes of this invention, the terms "expression construct," "expression vector," and "vector," are used interchangeably to demonstrate the application of the invention in a general, illustrative sense, and are not intended to limit the invention.

Thus, in another embodiment, an expression vector may be used to deliver an inhibitor of miR-21 to the muscle tissue of a subject. Numerous vectors are known in the art including, but not limited to, linear polynucleotides, polynucleotides associated with ionic or amphiphilic compounds, plasmids, and viruses. Examples of viral vectors include, but are not limited to, adenoviral vectors, adeno-associated virus vectors, retroviral vectors, and the like. An expression construct can be replicated in a living cell, or it can be made synthetically. An expression vector for expressing an inhibitor of miR-21 usually comprises a promoter operably linked to a polynucleotide encoding the inhibitory oligonucleotide. As used herein, the terms "operably linked" or "under transcriptional control" as referred to an oligonucleotide mean that a promoter is in the correct location and orientation in relation to the oligonucleotide to control the initiation of transcription by RNA polymerase and expression of said oligonucleotide. The term "promoter" refers to a DNA sequence recognized by the synthetic machinery of the cell, or introduced synthetic machinery, required to initiate the specific transcription of an oligonucleotide.

A second aspect of the present invention provides a pharmaceutical composition for use in the treatment of a MD and/or a MD-like myopathy comprising a miR-21 inhibitor as disclosed above. The pharmaceutical composition according to the invention thus comprises an effective amount of a miR-21 inhibitor together with pharmaceutically acceptable excipients and/or carriers. The pharmaceutical composition of the invention may also comprise one or more additional active ingredients.

In a particular embodiment, the pharmaceutical composition of the invention is for use in the treatment of a MD and/or MD-like myopathy, wherein the treatment comprises the regeneration of skeletal muscle. Preferred MDs to be treated by the pharmaceutical composition of the invention are DMD, Becker's muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy. In a preferred embodiment, the MD to be treated is DMD.

The miR-21 inhibitors of the present invention and pharmaceutical compositions comprising the same may be administered by any method known to those in the art suitable for delivery to the targeted organ, tissue, or cell type. For example, in certain embodiments of the invention, the composition may be administered by parenteral administration, such as intravenous injection, intraarterial injection, intrapericardial injection, or subcutaneous injection, or by direct injection into the tissue (e.g., intramuscular). In other embodiments, the pharmaceutical composition comprising miR-21 inhibitor may be administered by oral, transdermal, intraperitoneal, subcutaneous, sustained release, controlled release, delayed release, suppository, or sublingual routes of administration. The composition of the invention may also be administered by a catheter system.

In a prefered embodiment, the pharmaceutical composition of the invention is administered intramusculary is thus formulated in a form suitable for intra-muscular administration. Suitable formulations for intra-muscular administration include solutions, suspensions, dispersions, emulsions, oils and the like.

The pharmaceutical compositions comprising miR-21 inhibitor are formulated in conjuction with pharmaceutically acceptable excipients and/or carriers. The term "pharmaceutically acceptable" as used herein refers to compounds, materials, compositions, and/or dosage forms which are, within the scope of sound medical judgement, suitable for being administered to an animal, including a human, without undue toxicity, incompatibility, instability and allergic response, among others. Each pharmaceutically acceptable carrier, excipient, etc., must also be acceptable in the sense of being compatible with the other ingredients of the pharmaceutical formulation.

Pharmaceutically acceptable excipients and/or carriers used in the composition of the present invention are specifically suitable for gene therapy and preferably designed to enhance the stability and bioavailability of the miR-21 inhibitor.

In one embodiment, the composition of the invention comprises excipients and/or vehicles suitable for parental, preferably, intramuscular administration. Parenteral vehicles include sodium chloride solution, Ringer's dextrose, dextrose and sodium chloride, lactated Ringer's and fixed oils. Intravenous vehicles include fluid and nutrient replenishers, electrolyte replenishers such as those based on Ringer's dextrose, and the like. Examples are sterile liquids such as water and oils, with or without the addition of a surfactant and other pharmaceutically acceptable adjuvants. In general, water, saline, aqueous dextrose and related sugar solutions, and glycols such as propylene glycols or polyethylene glycol are preferred liquid carriers, particularly for injectable solutions.

The pharmaceutical compositions provided may be controlled-release compositions, i.e. compositions in which the active compound is released over a period of time after administration. For example, the agent may be administered using intravenous infusion, an implantable osmotic pump, a transdermal patch, liposomes, or other modes of administration. Alternatively, the composition is an immediate-release composition, i.e. a composition in which of the active compound is released immediately after administration.

The compositions also include, in another embodiment, incorporation of the active material into or onto particulate preparations of polymeric compounds such as polylactic acid, polglycolic acid, hydrogels, etc, or onto liposomes, microemulsions, micelles, unilamellar or multilamellar vesicles, erythrocyte ghosts, or spheroplasts.) Such compositions will influence the physical state, solubility, stability, rate of *in vivo* release, and rate of *in vivo* clearance. Liposomes including the miR-21 inhibitor are particularly suitable for the delivery of miR-21 inhibitor for use according to the present invention.

In one embodiment, the present invention comprises the use of a miR-21 inhibitor as a sole active ingredient for the treatment of a MD and/or a MD-like myopathy. However, also encompassed within the scope of the present invention is the use of a miR-21 inhibitor in combination with one or more additional therapeutic agents. In a particular embodiment, these additional agents are appropriate for treating MDs or MD-like myopathies. Preferably, the additional agents are designed for replacing or compensating for dystrophin or for ameliorating fibrosis. As will be apparent to the skilled in the art, the combination of a miR-21 inhibitor with one or more additional active ingredients may be effective not only when the active ingredients are used in a single pharmaceutical composition, but also when used in two different compositions, either administered simultaneously, sequentially or separately after a certain period of time. Furthermore, the skilled in the art will understand that the miR-21 inhibitor can be prescribed to be used together with the other active agent in order to treat a MD or MD-like myopathy. Throughout the description and claims the word "comprise" and variations of the word, are not intended to exclude other technical features, additives, components, or steps. Additional objects, advantages and features of the invention will become apparent to those skilled in the art upon examination of the description or may be learned by practice of the invention. The following examples and drawings are provided by way of illustration, and they are not intended to be limiting of the present invention. Furthermore, the present invention covers all possible combinations of particular and preferred embodiments described herein.

### BRIEF DESCRIPTION OF THE DRAWINGS

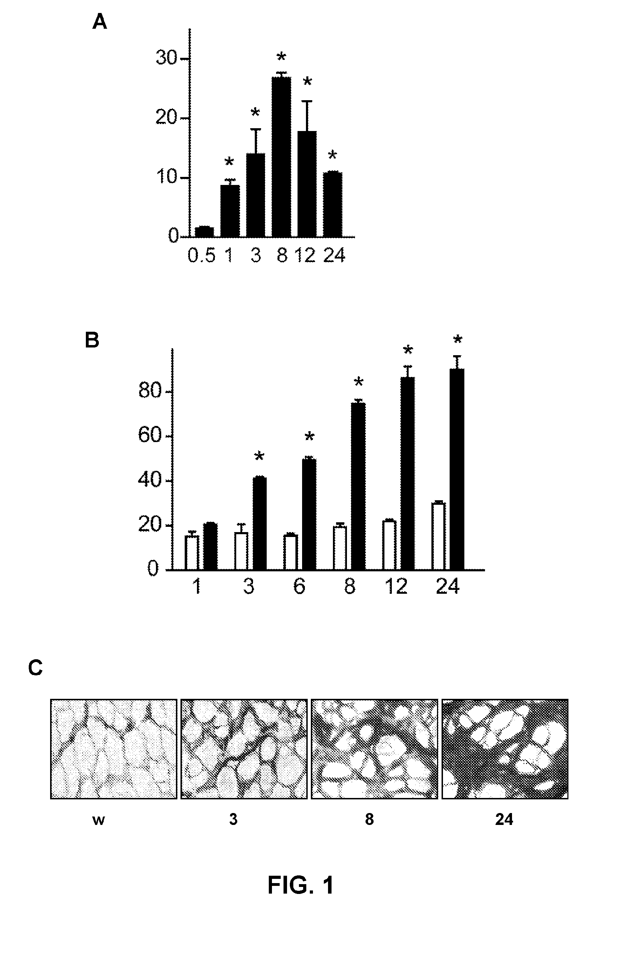

FIG. 1. A. miR-21 expression levels were analyzed in diaphragm muscle of 0.5, 1, 3, 8, 12 and 24 month-old *mdx* mice by qPCR. *mdx* mice values were normalized with respect to values of aged-matched wild type mice. (*P < 0.001 versus 0.5 months; means ± s.e.m; n=5, each group). B. Collagen content was analyzed in diaphragm muscle at the above indicated ages in *mdx* mice (black bars) and wild type mice (white bars) (*P < 0.001; means ± s.e.m; n=4, each group). C. Representative example of Sirius Red staining of diaphragm muscle sections: (w) 3 month-old wild type mice control, (3) *mdx* mice at 3 months, (8) 8 months, and (24) 24 months of age. Scale bar: 25 µm.

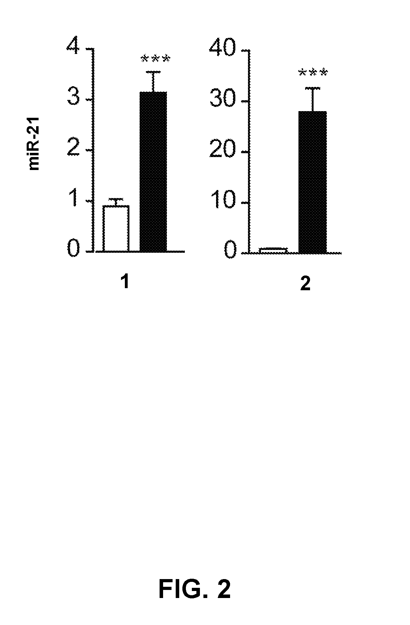

FIG. 2. Comparative qRT-PCR mRNA analysis of miR-21: (1) *mdx* and wild type mice, (2) DMD patients and healthy individuals. Wild type mice and healthy individuals are represented by the white bars while *dmx* mice and DMD patients are represented by black bars. (***P < 0.0001; means ± s.e.m; mouse n=5; n=7 patients (6-12 years of age), 6 healthy (9-15 years of age)).

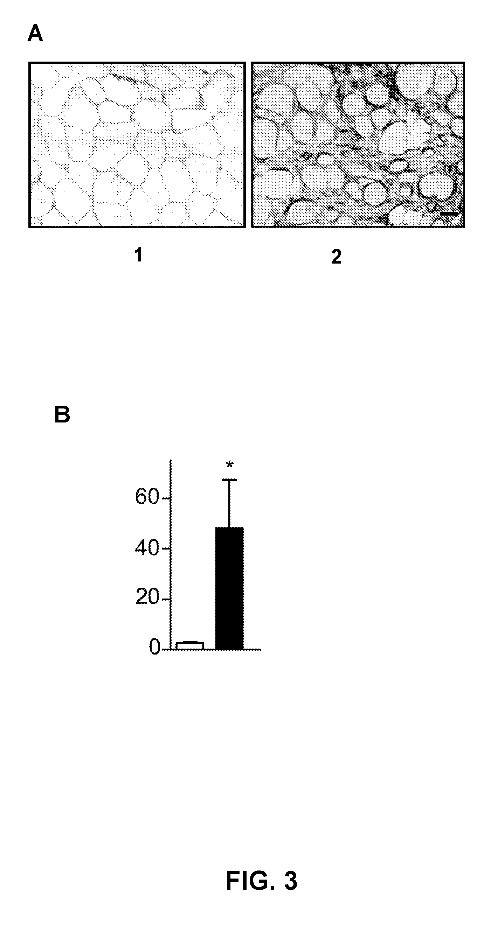

FIG. 3. A. Representative example of Sirius Red staining in sections of muscle biopsies: (1) healthy control subjects, and (2) DMD patients. Scale bar: 25 µm. B. Collagen accumulation in µg/mg protein in muscle biopsies of healthy controls and DMD patients. Healthy individuals are represented by the white bars while DMD patients are represented by black bars. (*P < 0.01; means ± s.e.m; n:=7 patients (6-12 years of age), 6 healthy (9-15 years of age)).
FIG. 4. Efficacy of miR-21 silencing in treating muscular dystrophy symptoms. A. Sirius Red staining of *gastrocnemius* muscle sections from young *mdx* mice (3 months-old) after 1 month administration of (1) scramble control oligomiR or (2) miR-21 mimic. Scale bar: 25 µm. B. Sirius Red staining of *gastrocnemius* muscle sections from 24 month-old *mdx* mice after 1 month administration of (3) scramble control oligomiR or (4) anti-miR-21. Scale bar: 25 µm. C. (a) Collagen accumulation and (b) miR-21 expression in *gastrocnemius* muscle after 1 month administration of anti-miR-21, miR-21 mimic or scramble control oligomiR: (W) wild type, (1) young *mdx* + scramble, (2) young *mdx* + miR-21 mimic, (3) aged *mdx* + scramble, and (4) aged *mdx* + anti-miR-21 (*P < 0.001; means ± s.e.m; n=5, each group).
FIG. 5. Efficacy of miR-21 silencing in improving the regeneration of dystrophic muscles. A. H/E staining of *gastrocnemius* muscle sections from 24 month-old *mdx* mice treated for 1 month with (1) scramble control oligomiR or (2) anti-miR-21. Scale bar: 50 µm. B. Grip strenght peak force (N) measured during 10 days (t) in 24 month-old *mdx* mice after treatment with scramble control oligomiR (●) or anti-miR-21 (■). C. Grip strenght peak force average (N) measured in 24 month-old *mdx* mice after 1 month treatment with scramble control oligomiR (white bar) or anti-miR-21 (black bar).

### EXAMPLES

The following examples are provided by way of illustration, and are not intended to be limiting of the present invention.

### Materials and methods

### 1. Animal model.

Wild-type (C57BL/6) and *mdx* C57BL/6 mice were purchased from The Jackson Laboratory (Bar Harbor, ME, USA). Mice were housed in the animal facility of the PRBB Barcelona under conventional conditions with constant temperature and humidity and fed a standard diet. The experiments were mainly performed with the *mdx* C57BL/6 mouse, one of the most widely used model of muscular dystrophy. In some experiments wild type (WT) C57BL/6 mouse were used as internal further control. All animal experiments were approved by the Catalan Government Animal Care Committee.

### 2. DMD patient study.

Specimen preparation for histopathological examination was performed with DMD patients (7 patients between 6 to 12 years of age) and healthy human controls of similar age (9 to 15 years). Muscle samples were obtained by standard quadriceps muscle biopsy.

### 3. miRNA treatment in vivo.

5 µg of either anti-miR-21, miR-21 mimic or a scramble control oligomiR (Dharmacon), disolved in 10 µl of saline and were injected intramuscularly into *tibialis anterior* or *gastrocnemius* muscles of *mdx* mice. anti-miR-21 is a shRNA oligonucleotide designed to inhibit a miRNA having miR-21 sequence (SEQ ID NO: 1). Thus the inhibitor contains the perfect complementary of this sequence. miR-21 mimic is an oligonucleotide which highly resembles miR-21 and performs miR-21 functions. Scramble control oligomiR is a shRNA oligonucleotide comprising a nonsense sequence such that it does not inhibit miR-21 nor any other known sequence. Of note: mature mouse and human miR-21 sequences have 100% identity. for Morphological examinations were performed at the indicated days after injury/treatment.

### 4. Histology.

*Tibialis anterior, gastrocnemius* or dyaphragm muscles were isolated and frozen in liquid nitrogen-cooled isopentane or embedded in paraffin. Cryosections (10 µm thickness) were stained with Hematoxilin/eosin (H/E, SIGMA) or Sirius Red (Sigma). Digital images were acquired with a microscope DMR6000B (Leica) equipped with a DFC300FX camera for immunohistochemical color pictures, using Plan Fluo 10x/0.3 and 20x/0.75 objective lens and maximum optical sections. Images were composed and edited in Photoshop CS4 (Adobe), where background was reduced using brightness and contrast adjustments applied to the whole image. Image analysis of fibrotic tissue was performed with the public domain computer-assisted image analysis software ImageJ (NIH, USA). Fiber size measurements were performed as described (Suelves M, et al. "uPA deficiency exacerbates muscular dystrophy in MDX mice". J Cell Biol 2007, vol. 178, p. 1039-1051).

### 5. Quantification of miRNA expression.

The total RNA including the miRNA fractions was isolated using miRNeasy kit (Qiagen, ref. 217004) following manufacturer instructions. The quantification of miRNA-21 was performed by quantitative reverse transcription PCR (qRT-PCR) using Taqman assays for miRNA (Applied Byosistems mmu-miR-21, ref. 002493) and Taqman Fast Universal Master Mix (Applied Byosistems, ref. 4352042) following manufacturer instructions. miRNA levels were quantified using miR-U6B as the house-keeping miRNA.

### 6. Characterization of muscle fibrosis.

Biochemical quantification of collagen content in muscle was performed according to (Lopez-De Leon A and Rojkind M. "A simple micromethod for collagen and total protein determination in formalin-fixed paraffin-embedded sections". J Histochem Cytochem 1985, vol. 33, p. 737-743). Histological characterization of muscle fibrosis after Sirius Red staining was also done by image analysis quantification as described above.

### 7. Grip strength assay.

Forelimb grip strength in 24 month-old *mdx* mice after 1 month treatment with Scramble control oligomiR or anti-miR-21 was measured as tension force (in newton, N) using a computerized force transducer (Grip Strength Meter, Bioseb). In each trial, the mouse was allowed to grasp a metal rod connected to the machine, and the researcher slowly pulled the mouse by the tail until the digital gauge recorded the peak tension produced. Three trials of 10 measurements per trial were performed for each animal with a few minutes resting period between trials. The same measurements were repeated on the next days for ten days, and the highest value of each experiment was used. The average tension force (newton, N) for each group of mice was provided by the transducer.

### 8. Statistical analysis.

Graphpad Prism software was used for all statistical analyses. Results from corresponding time points of each group were averaged and used to calculate descriptive statistics. One-way ANOVA and Tukey post-hoc test, or Dunnett's post-hoc test were used on multiple comparisons and all possible pair wise comparisons among groups at each time point. Data are mean ± s.e.m. Significance was accepted at P≤0.05.

### Results

The following results demonstrate that miR-21 is involved in the regulation of the process of muscle degeneration/regeneration during the onset and course of MD. It has also been found that inhibition of miR-21 by means of interference RNA technology favours tissue regeneration as well as ameliorates fibrotic process in an animal model of DMD.

### 1. miR-21 is up-regulated in MD (mdx mouse and DMD patients)

The expression of miR-21 was found dysregulated (increased) in dystrophic muscle of human DMD patients and of the *mdx* mice, the animal model of DMD (see FIG. 1 A and FIG 2). Additionally, elevated miR-21 expression in muscle tissue parallels collagen production in *mdx* mice and dystrophic muscle of human DMD patients (FIG. 1 B-C and FIG. 3), thus indicating advanced muscle disease.

### 2. Inhibition of miR-21 improves regeneration in mdx and reduces fibrosis

As a result of extensive research the inventors have found that miR-21 is a good target for therapeutic intervention to enhance muscular tissue repair, especially in long-term severe myopathies. Thus, modulation of miR-21 can be an effective therapy for DMD patients.

Consistent with this, treatment with anti-miR-21, but not with scramble oligomiR, reversed fibrosis in limb muscles of 24 month-old *mdx* mice (at stage at which *mdx* fibrosis is generally considered irreversible) (see FIG. 4B and C). Conversely, the sole overexpression of miR-21 by intramuscular administration of a miR-21 mimic anticipated and exacerbated fibrosis in young *mdx* mice (3 month-old) (FIG. 4 A and C). These results demonstrate the efficacy of miR-21 silencing in preventing and treating DMD. Notably, miR-21 interference for 1 month in very old *mdx* dystrophic mice also reduced muscle degeneration (FIG. 5 A). The figure shows not only attenuation of fibrosis but, more importantly, an unexpected recovery of muscle fiber fitness.

Of note, treatment with anti-miR-21 also protected *mdx* mice from functional deterioration, as demonstrated by the increased force elicited by treated limbs using a grip strength test, an indicator of better motor function compared with controls (FIG. 5 B-C).

Since affected individuals with prominent muscle damage at advanced disease stages of DMD represent the vast majority of patients, and no treatment for efficiently enhancing muscle regeneration and reducing muscle fibrosis is yet known, these results have undoubtedly a strong therapeutic potential.

## Claims

1. A miRNA-21 (miR-21) inhibitor for use in the treatment of a muscular dystrophy and/or a muscular dystrophy-like myopathy.

2. The miR-21 inhibitor for use according to claim 1, wherein the treatment comprises the regeneration of skeletal muscle.

3. The miR-21 inhibitor for use according to any of the claims 1-2, wherein the muscular dystrophy is selected from the group consisting of Duchenne muscular dystrophy, Becker's muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy.

4. The miR-21 inhibitor for use according to claim 3, wherein the muscular dystrophy is Duchenne muscular dystrophy.

5. The miR-21 inhibitor for use according to any of the claims 1-4, wherein the miR-21 inhibitor is an agent that prevents or reduces miR-21 transcription.

6. The miR-21 inhibitor for use according to any of the claims 1-4, wherein the miR-21 inhibitor is an agent that prevents or reduces miR-21 regulatory activity.

7. The miR-21 inhibitor for use according to any of the claims 5-6, wherein the miR-21 inhibitor is selected from the group consisting of an inhibitory oligonucleotide, a ribozyme, a protein, a small molecule chemical compound, and a combination thereof.

8. The miR-21 inhibitor for use according to claim 7, wherein the miR-21 inhibitor is an inhibitory oligonucleotide selected from the group consisting of a small single stranded RNA, a small hairpin RNA (shRNA) and a small interference RNA (siRNA).

9. The miR-21 inhibitor for use according to claim 8, wherein the miR-21 inhibitor is a small hairpin RNA.

10. A pharmaceutical composition for use in the treatment of a muscular dystrophy and/or a muscular dystrophy-like myopathy, comprising a miR-21 inhibitor as defined in any of the claims 5-9 along with pharmaceutically acceptable excipients and carriers.

11. The pharmaceutical composition for use according to claim 10, wherein the treatment comprises the regeneration of skeletal muscle.

12. The pharmaceutical composition for use according to any of the claims 10-11, wherein the muscular dystrophy is selected from the group consisting of Duchenne muscular dystrophy, Becker's muscular dystrophy, limb girdle muscular dystrophy, congenital muscular dystrophy, facioscapulohumeral muscular dystrophy, myotonic muscular dystrophy, oculopharyngeal muscular dystrophy, distal muscular dystrophy, and Emery-Dreifuss muscular dystrophy.

13. The pharmaceutical composition for use according to claim 12, wherein the muscular dystrophy is Duchenne muscular dystrophy.

14. The pharmaceutical composition for use according to any of the claims 10-13, which is for parenteral administration.

15. The pharmaceutical composition for use according to claim 14, which is for intramuscular administration.
